# EUROPEAN PATENT SPECIFICATION

(11) **EP 2 397 168 B1**
(45) Date of publication and mention of the grant of the patent: **07.05.2014**
(21) Application number: 11170464.9
(22) Date of filing: 18.06.2011
(51) Int. Cl.: A61M 1/36

(54) **Dialysis machine comprising a cassette unit**
Dialysemaschine mit einer Kassetteneinheit
Machine de dialyse avec une cassette

(30) Priority: 18.06.2010 IT BO20100393
(43) Date of publication of application: 21.12.2011
(73) Proprietor: Bellco S.r.l., Mirandola (IT)
(72) Inventor: Cianciavicchia, Domenico, 64040 CAVUCCIO (IT); Fiorenzi, Andrea, 41013 CASTELFRANCO EMILIA (IT); Aldrovandi, Mauro, 41037 MIRANDOLA (IT)
(74) Representative: Bergadano, Mirko

(56) References cited:
- WO-A1-2004/000391
- WO-A1-2007/133259
- GB-A- 2 310 616
- US-A- 3 946 731

## Description

The present invention relates to a dialysis machine comprising an improved-type cassette unit.

Dialysis machines generally comprise a cassette serving the function of ensuring a given order to the arrangement of the tubes used to carry the various liquids involved in the dialysis treatment.

The cassettes are components of the dialysis machine which are physically separate from the main body of the machine itself, and are fitted in use on a front panel of the main body itself. The cassettes are generally made of rigid, transparent plastic material, such as PVC, for example.

The tubes related to the venous line, to the arterial line of blood, and to other liquid lines involved in the dialysis treatment converge into the cassette.

With this regard, the cassettes may be internally split up into a plurality of compartments, fluid-tightly separate from one another, each of which is arranged to accommodate a particular fluid of the dialysis treatment.

The above-described cassettes further comprise a pressure measuring device for each compartment. The pressure measuring device usually consists of a measuring tube having a first end facing the interior of the compartment to be measured and a pressure sensor arranged inside the main body of the machine and to which a second end of the measuring tube is connected.

In order to avoid any malfunction and/or contamination of the pressure sensor, the end of the tube facing the interior of the compartment of the venous line must be kept constantly and appropriately spaced apart from the free blood surface. Indeed, if the end of the tube and the free blood surface were not kept at an appropriate distance from each other, simple blood level variations could make the blood enter into the measuring tube and reach the pressure sensor.

In order to avoid minor blood level variations from causing the above problems, the venous line compartment is shaped so that the free blood surface occupies a sufficiently wide space so that a blood volume variation in the compartment itself is solved in a minor level variation.

Although this solution ensures that the blood does not come in contact with the tube, it suffers however from the problem related to a wide contact surface between blood and air, resulting in the risk of surface clotting of the blood and possible contaminations thereof.

In order to obviate this problem, the free surface of the blood must be kept in a constant movement and the blood must be periodically replaced within the venous line compartment, during which operation the dialysis treatment must be interrupted.

Document WO 2004/000391 A1 discloses a dialysis machine having a blood reservoir and one level sensor that can optionally be arranged e.g. in a section of a service line.

Document GB 2 310 616 A discloses a dialysis machine having a blood reservoir and two blood level sensors, that allow maintaining the blood level in the blood reservoir between a maximum and a minimum level.

It is an object of the present invention to provide a cassette unit, the technical features of which overcome the prior art drawbacks.

It is the object of the present a cassette unit, the features of which are set forth in claim 1, and the preferred and/or auxiliary features of which are set forth in claims 2-5.

For a better understanding of the invention, an embodiment will be described below by way of mere non-limitative example, with the aid of the figures of the accompanying drawing, in which:
figure 1 is a front view of a dialysis machine object of the present invention; and
figure 2 shows an enlargment of the cassette unit of the machine in figure 1 with parts removed for clarity.

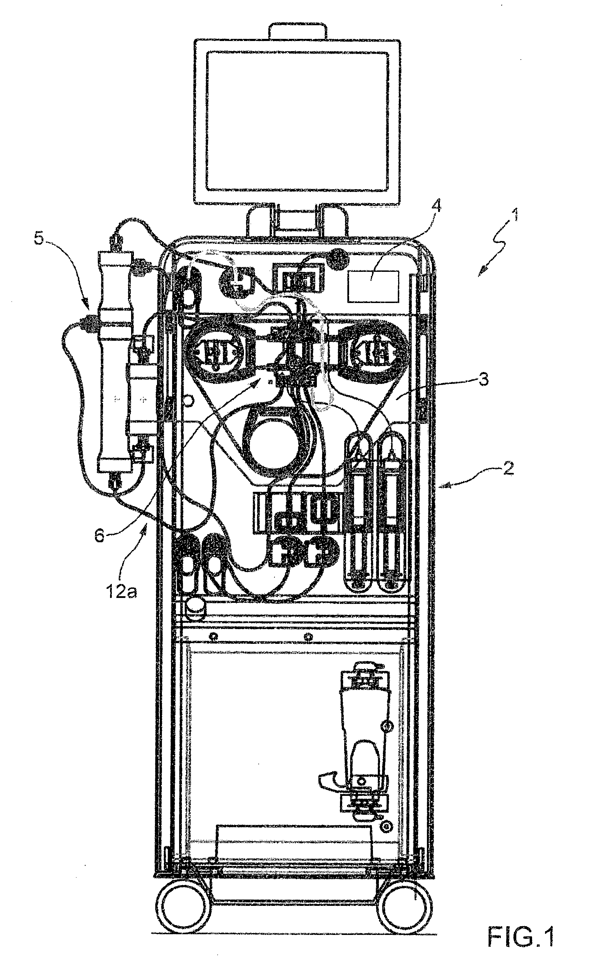

In figure 1, numeral 1 indicates as a whole the dialysis machine object of the present invention.

Machine 1 comprises a main body 2 where the devices required for the specific dialysis treatment are accommodated, such as for example pumps and an electronic control unit 4 (diagrammatically shown), covering panels (only front panel 3 is shown in the figure), a dialysis filter 5 and a cassette unit 6.

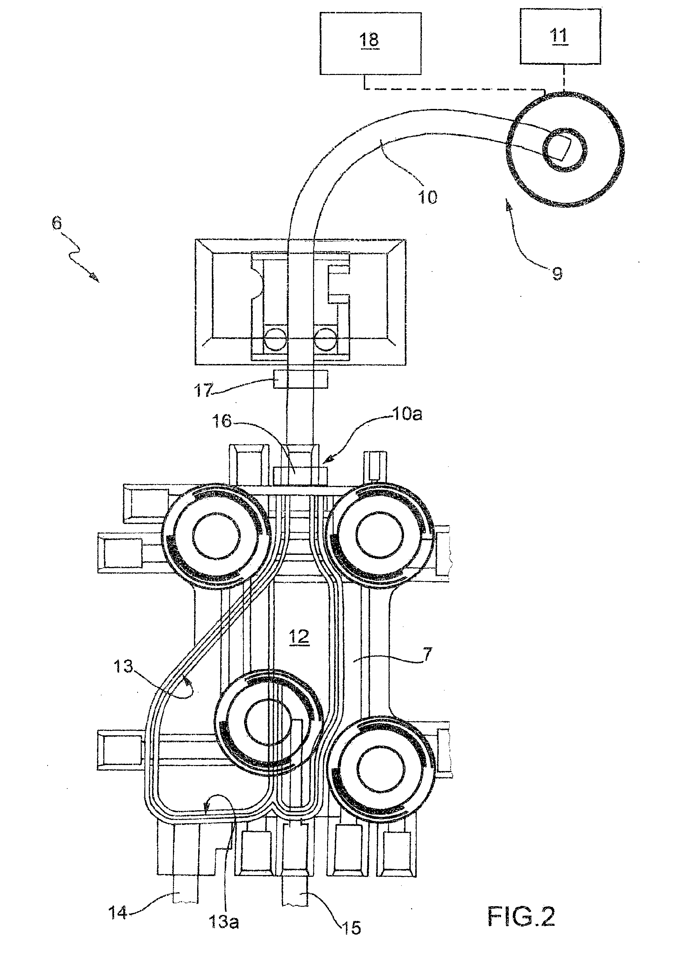

As shown in figure 2, cassette unit 6 comprises a cassette 7, to which a plurality of tube used for carrying the various dialysis treatment fluids are connected, and a pressure measuring device 9. The pressure measuring device 9 comprises, in turn, a measuring tube 10 extending from the front panel 3, and a pressure sensor 11 (only diagrammatically shown) arranged inside machine 1. The measuring tube 10 is connected to the pressure sensor 11 for measuring the pressure of the compartment where it faces.

Cassette 7 is secured to the front covering panel 3 by means of a releasable fastening device (known and not shown for simplicity).

A blood compartment 12 of a venous line 12a is obtained within cassette 7. Blood compartment 12 is defined by a plurality of walls 13, and measuring tube 10 has a first end 10a thereof facing on top the blood compartment 12.

Moreover, the open ends of a loading tube 14 and of an unloading tube 15, through which the blood respectively enters and exits to/from the blood compartment 12 itself, face a bottom wall 13a of blood compartment 12. Feeding from the bottom facilitates the operation of degassing the blood before it is introduced back into the patient.

Cassette unit 6 comprises a pair of level sensors 16 and 17 (diagrammatically shown), arranged at a connection between measuring tube 10 and cassette 7, and along the measuring tube 10 which extends outwards from the front covering panel 3.

In particular, level sensor 16 is of the optical or capacitance type, while level sensor 1 is of the ultrasound type.

In use, level sensor 16 detects the complete filling of blood compartment 12, while level sensor 17 detects the increase of blood level along measuring tube 10.

Cassette unit 6 further comprises a peristaltic pump 18 (only diagrammatically shown) accommodated within the main body 2, which is connected to measuring tube 10. The peristaltic pump 18 serves the function of insufflating or taking air into the measuring tube 10 to produce a blood level variation in the measuring tube 10 itself.

In machine 1, the control unit 4 is connected to the various devices for adjusting their operation. In particular, control unit 4 is connected to the two level sensors 16 and 17 and to the peristaltic pump 18. Once level sensor 17 has detected the presence of blood within the measuring tube 10, it sends a signal to the control unit 4, which in turn controls pump 18 to insufflate air into the measuring tube 10 to lower the blood level and thus return it within safety limits.

Moreover, control unit 4 may control pump 18 to perform periodic insufflation/suction cycles to move the free surface of the blood so as to provide a further guarantee to avoid clot formation.

The presence of the two level sensors 16 and 17 and the connection of the measuring tube 10 to the peristaltic pump 18, allows the dialysis treatment to be carried out while keeping the blood compartment 12 completely full of blood, instead of only partially filled as occurs in the prior art machines. Such a possibility ensures that the space occupied by the free surface of the blood is very small, i.e. equal to the area of the cross section of measuring tube 10. Thereby, the contact surface between blood and air is drastically reduced and so is the possibility of contamination and clotting.

As apparent from the above description, the machine object of the present invention allows the contact surface between blood and air to be drastically reduced, and thus the risk of clotting phenomena are also reduced without any need to interrupt the dialysis treatment and without hazarding the operation and integrity of the pressure sensor.

Moreover, the position of level sensor 17 along the tube may be varied according to the specific needs of the single machine and of the single treatment.

## Claims

1. A dialysis machine (1) comprising a dialysis filter (5), an electronic control unit (4), and a cassette unit (6); said cassette unit (6) comprising a cassette (7) in which a blood compartment (12) of a venous line (12a) is defined, at least one pressure measuring device (9) comprising a pressure sensor (11) and a measuring tube (10) facing on top the blood compartment (12), and a pump (18) connected to said measuring tube (10), and a first level sensor (16); said dialysis machine being **characterized in that**
the first level sensor (16) being arranged at a joint of the measuring tube (10) to the cassette (7) and adapted to detect the filling of the blood compartment (12), and said cassette unit (6) comprises a second level sensor (17) arranged at a segment of the measuring tube (10) so as to detect the presence of blood therein; said pressure sensor (11), said pump (18) and said level sensors (16, 17) being connected to said electronic control unit (4).

2. The dialysis machine according to claim 1, **characterized in that** said first level sensor (16) is of the optical type or of the capacitive type.

3. The dialysis machine according to claim 1 or 2, **characterized in that** said second level sensor (17) is of the ultrasound type.

4. The dialysis machine according to one of the preceding claims, **characterized in that** said pump (18) performs periodic cycles of insufflation/suction to move the free surface of the blood.

## Patentansprüche

1. Dialysemaschine (1), die einen Dialysefilter (5), eine elektronische Steuereinheit (4) und eine Kassetteneinheit (6) umfasst; wobei die Kassetteneinheit (6) eine Kassette (7), in der ein Blutfach (12) einer venösen Leitung (12a) definiert ist, wenigstens eine Druckmessvorrichtung (9) mit einem Drucksensor (11) und einem Messrohr (10), das dem Blutfach (12) von oben zugewandt ist, eine Pumpe (18), die mit dem Messrohr (10) verbunden ist, und einen ersten Pegelsensor (16) enthält; wobei die Dialysemaschine **dadurch gekennzeichnet ist, dass** der erste Pegelsensor (16) an einer Verbindung des Messrohrs (10) mit der Kassette (7) angeordnet ist und dafür ausgelegt ist, die Befüllung des Blutfachs (12) zu detektieren, und die Kassetteneinheit (6) einen zweiten Pegelsensor (17) umfasst, der an einem Segment des Messrohrs (10) so angeordnet ist, dass er das Vorhandensein von Blut darin detektiert; wobei der Drucksensor (11), die Pumpe (18) und die Pegelsensoren (16, 17) mit der elektronischen Steuereinheit (4) verbunden sind.

2. Dialysemaschine nach Anspruch 1, **dadurch gekennzeichnet, dass** der erste Pegelsensor (16) vom optischen Typ oder vom kapazitiven Typ ist.

3. Dialysemaschine nach Anspruch 1 oder 2, **dadurch gekennzeichnet, dass** der zweite Pegelsensor (17) vom Ultraschalltyp ist.

4. Dialysemaschine nach einem der vorhergehenden Ansprüche, **dadurch gekennzeichnet, dass** die Pumpe (18) periodische Insufflations-/Saugzyklen ausführt, um die freie Oberfläche des Blutes zu bewegen.

## Revendications

1. Machine de dialyse (1) comprenant un filtre de dialyse (5), une unité de commande électronique (4), et une unité de cassette (6) ; ladite unité de cassette (6) comprenant une cassette (7) dans laquelle est défini un compartiment du sang (12) d'une ligne veineuse (12a), au moins un dispositif de mesure de pression (9) comprenant un capteur de pression (11) et un tube de mesure (10) regardant la partie supérieure du compartiment du sang (12), et une pompe (18) reliée audit tube de mesure (10) ; et un premier capteur de niveau (16) ; ladite machine de dialyse étant **caractérisée en ce que** le premier capteur de niveau (16) est agencé au niveau d'une jonction du tube de mesure (10) à la cassette (7) et adapté pour détecter le remplissage du compartiment du sang (12), et ladite unité de cassette (6) comprend un deuxième capteur de niveau (17) agencé au niveau d'un segment du tube de mesure (10) de manière à y détecter la présence de sang ; ledit capteur de pression (11), ladite pompe (18) et lesdits capteurs de niveau (16, 17) étant reliés à ladite unité de commande électronique (4).

2. Machine de dialyse selon la revendication 1, **caractérisée en ce que** ledit premier capteur de niveau (16) est du type optique ou du type capacitif.

3. Machine de dialyse selon la revendication 1 ou 2, **caractérisée en ce que** ledit deuxième capteur de niveau (17) est du type à ultrasons.

4. Machine de dialyse selon l'une quelconque des revendications précédentes, **caractérisée en ce que** ladite pompe (18) exécute des cycles périodiques d'insufflation/aspiration pour déplacer la surface libre du sang.
